# EUROPEAN PATENT APPLICATION

(11) **EP 3 943 061 A1**
(43) Date of publication of application: **26.01.2022**
(21) Application number: 20187220.7
(22) Date of filing: 22.07.2020
(51) Int. Cl.: A61K 6/54, A61K 6/62, A61K 6/69, A61K 6/891, A61L 27/44, A61L 27/54

(54) **BIOCOMPOSITE MATERIAL FOR ENDODONTIC APPLICATION**

(71) Applicant: Khalil, Wael Hassan, Beirut (LB)
(72) Inventor: Khalil, Wael Hassan, Beirut (LB)
(74) Representative: Viering, Jentschura & Partner mbB Patent- und Rechtsanwälte

(57) **Abstract**

The present invention relates to a biocomposite material comprising at least one biopolymer matrix component and at least one bioactive filler component, to a method of manufacturing thereof, a method of hardening thereof, as well as to the use of said biocomposite material in biopulpectomy procedures for temporary or permanent teeth, in direct or indirect pulp capping, as a retrograde filling material for endodontic surgery, or for tooth or root perforation.

## Description

### FIELD OF THE INVENTION

The technical field generally relates to composite material. In particular, the present disclosure is concerned with composite material promoting dentin (or dental structure) and pulp regeneration, such as used in pulp capping or biopulpotomy or tooth perforation or also as retrograde filling material in endodontic surgery.

### BACKGROUND OF THE INVENTION

Root canal treatment (also known as endodontic therapy, endodontic treatment, or root canal therapy) is a treatment sequence for the inflamed, infected or necrotic pulp of a tooth, in which the suffering pulp cannot be treated otherwise successfully. Root canal treatment can also be applied as preventive management before crowning tooth or sometime in some particular case related to surgical procedure in the jaw in proximity to tooth apex.

Biopulpectomy (complete removal of the pulp) refers to root canal treatment when the pulp of the tooth is still vital and it involves the removal of these structures, disinfection and the subsequent shaping, cleaning, and decontamination of the hollows with small files and irrigating solutions, and the obturation (filling) of the decontaminated canals. An alternative to biopulpectomy is biopulpotomy (partial removal or resection of the pulp), which refers to removing the pulp chamber (the coronal part of the pulp without touching the pulp inside the canal (in the root)), but due to weak prognosis of biopulpotomy technique (compared to biopulpectomy) this technique usually is limited to temporary teeth because the life span of temporary teeth is limited to a few years only. The biopulpotomy technique as mentioned consists of removing the pulp chamber without touching or exploring the radicular part of the pulp, and in almost the majority of cases using fixating agents for the remaining part of the pulp (root pulp) without hoping pulp or dentine regeneration.

In some other cases, the decay is very deep and close to the pulp but the tooth is still vital with 'healthy pulp', so in order to save the pulp and spare it a pulp capping is applied, it is called direct capping if the pulp is exposed during decay removal and indirect capping, if a thin layer of dentin is still present between the cavity and the pulp. In the case of direct pulp capping, we hope having dentin regeneration of the exposed part of the pulp. The advantage of pulp capping (direct and indirect) is to preserve the pulp without wishing to have pulp necrosis. The limitation of this technique is its poor prognosis and the high risk of failure and non-negligible cases we can end up having immediate pulp inflammation (pulpitis) or delayed pulp necrosis due to pulp reaction toward the technique or toward the material used that is closed to the pulp and it should be noted that pulp capping is only indicated if the tooth was vital, asymptomatic and with no sign of irreversible pulpitis.

In summary, the technique consists of putting special material (that could promote dentin regeneration) on the exposed pulp and then a filling restoration is placed on the top.

Whereas for tooth perforation (floor or root) it could be iatrogenic or due to extended caries in both cases the obturation of this perforation is not all the time guaranteed or successful due to difficulties in using adequate material and in handle and manipulate it.

Whilst current materials have not achieved considerable success in terms of pulp and dentin regeneration in pulp capping, in biopulpotomy procedure or in tooth perforation, there remains an ongoing need for improved materials that are biocompatible, promote healing of the remaining part of the pulp, or capable of promoting dentin or dental structures regeneration, able to isolate pulp from filling efficiently and to prevent pulp necrosis. And spare the tooth from root canal treatment that lead to additional cost and time and complication to the patient.

Thus, it is desired to provide composite material that allows at least some of the aforementioned objectives to be achieved.

### SUMMARY OF THE INVENTION

This need is met by the products, uses and further embodiments of the present invention in providing for composite material named biocomposite that may be hardened after manipulation and placement close to the pulp or directly on exposed or partially excised pulp to promote pulp (nerve and vessels) and dentin regeneration.

In a first aspect, the present invention thus relates to a biocomposite material comprising at least one biopolymer matrix component and at least one bioactive filler component.

In a second aspect, the present invention also relates to a method for the manufacturing of a biocomposite material, as herein defined, comprising the step a) of mixing together the at least one bioactive filler component, preferably in particulate form, and the at least one biopolymer matrix component, preferably in liquid form.

In a third aspect, the present invention further relates to a method for obtaining a hardened biocomposite material, the method comprising the steps of:
i) providing a biocomposite material, as herein disclosed and defined, or obtainable in a method, as herein described; and
ii) exposing said biocomposite material to electromagnetic radiation, preferably Ultraviolet (UV), more preferably UVA, more preferably UV having a wavelength of about 365 nm to obtain a hardened biocomposite material.

In a fourth aspect, the present invention relates to the use of a biocomposite material, as herein defined, or obtainable in a method , as herein defined, in biopulpectomy procedures for temporary or permanent teeth, in direct or indirect pulp capping, as a retrograde filling material for endodontic surgery, or for tooth or root perforation.

Finally, in a fifth aspect, the present invention relates to a kit-of-parts, comprising at least one biopolymer matrix component, as herein defined, at least one bioactive filler component, as herein defined, and optionally further at least one UV light source and/or at least one opaque, preferably sealable container suitable for accommodating a biocomposite material, as herein defined, or a biocomposite material obtainable in a method, as herein defined.

These and other aspects of the invention will be apparent from and elucidated with reference to the embodiment(s) described hereinafter as well as the dependent claims.

### BRIEF DESCRIPTION OF THE DRAWINGS

**Figure 1****:** -number 1 represents coronal part of the pulp; -number 2 represents the radicular part of the pulp.
**Figure 2****:** -number 3 represents the cavity of caries removed and after partial excision of the pulp = biopulpotomy (H3 =handpiece).
**Figure 3****:** -number 4 represents the biocomposite material placed in the base of the cavity; -number 5 represents UV light and H5 the UV source.

### DETAILED DESCRIPTION OF THE INVENTION

The following specific description is merely exemplary in nature and is not intended to limit the application and uses. Furthermore, there is no intention to be bound by any expressed or implied theory presented in the preceding technical field, background, brief summary or the following detailed description.

Unless otherwise defined, all technical and scientific terms used herein have the same meaning as commonly understood by one of ordinary skill in the art.

In the present specification, the terms "a" and "an" and "at least one" are the same as the term "one or more" and can be employed interchangeably.

"One or more", as used herein, relates to at least one and comprises 1, 2, 3, 4, 5, 6, 7, 8, 9 or more of the referenced species. Similarly, "at least one" means one or more, i.e. 1, 2, 3, 4, 5, 6, 7, 8, 9 or more.

"At least one", as used herein in relation to any component, refers to the number of different entities, such as chemically different molecules, i.e. to the number of different types of the referenced species, but not to the total number of entities (such as molecules).

Numeric values specified without decimal places here refer to the full value specified with one decimal place, i.e., for example, 99 % means 99.0 %, unless otherwise defined.

"About" and "approximately", as used herein in relation to a numerical value, refer to said value ±10%, preferably ±5%.

All percentages given herein in relation to the compositions or formulations relate to weight % relative to the total weight of the respective composition or formula, if not explicitly stated otherwise.

The term "liquid", as used herein, refers to compounds or mixtures of compounds that are flowable and pourable at room temperature (about 15 °C to about 25 °C).

According to the present invention, the composite material comprises at least one biopolymer matrix component, as herein defined and described below, and at least one bioactive filler component, as herein described and defined below.

In various embodiments, the biopolymer matrix component comprises or consists of a triacrylate. In various embodiments, the biopolymer matrix component comprises or consists of at least one thiol-triacrylate.

In various embodiments, the thiol-triacrylate may promote dentine regeneration. Methods for providing a thiol-triacrylate polymer to be comprised in or constitute the biopolymer matrix component of the biocomposite material of the present invention are generally known in the art and may comprise reacting at least one triacrylate monomer, at least one thiol-ene crosslinking agent and at least one catalyst.

In various embodiments, the biopolymer matrix component comprises at least one triacrylate monomer. Triacrylate monomers suitable for employment in the context of the present invention include, but are not limited to, TMPTA (trimethylolpropane triacrylate), PETA (pentaerythritol triacrylate), and derivatives of TMPTA including, without limitation, TMPeTA (trimethylolpropane ethoxylate triacrylate) and TMPPTA (trimethylolpropane propoxylate triacrylate).

Thus, according to some embodiments, the at least one triacrylate monomer is selected from the group consisting of TMPTA (trimethylolpropane triacrylate), PETA (pentaerythritol triacrylate), TMPeTA (trimethylolpropane ethoxylate triacrylate), and TMPPTA (trimethylolpropane propoxylate triacrylate).

In various embodiments, the biopolymer matrix component comprises at least one thiol-ene crosslinking reagent. Thiol-ene crosslinking agents suitable for employment in the context of the present invention are generally known in the art. A non-limiting example of thio-ene crosslinking reagents suitable for employment in the context of the present invention is triacrylate-trimethylolpropane-tris-(3-mercaptopropionate) (TMPTMP). According to various embodiments, the thiol-ene crosslinking reagent is triacrylate-trimethylolpropane tris (3-mercaptopropionate) (TMPTMP).

In various embodiments, the biopolymer matrix component comprises at least one catalyst. Catalyst suitable for employment in the context of the present invention are generally known in the field and chosen accordingly. Non-limiting examples of catalysts suitable for employment in the context of the present invention include diethylamine (DEA). According to various embodiments, the at least catalyst is diethylamine.

In various embodiments, the biopolymer matrix component is prepared by mixing at least one triacrylate monomer with the thiol-en crosslinking reagent and the catalyst.

Thioacrylate can be prepared by adding the thiol-ene crosslinking reagent, which preferably is TMPTMP, to a triacrylate monomer such as PETA, TMPTA or TMPeTA, preferably in 1:1 molar ratio. A catalyst, preferably DEA, is added in an amount in the range of about 2 to 16 mol%.

In various embodiments, the biopolymer matrix component comprises triacrylate TMPTA, TMPTeA or PETA.

According to various embodiments, the amount of triacrylate may be in the range of about 10 to about 90 vol.-%, preferably in the range of about 20 to about 80 vol.-%, more preferably in the range of about 30 to about 70 vol.-%, even more preferably in the range of about 40 to about 60 vol.-%, such as bout 40, 45, 50, 55 or 60 vol.-%, based on the total volume of the biopolymer.

In various embodiments, the biocomposite material comprises a biopolymer thiotriacrylate. The amount of biopolymer thiotriacrylate may be in the range of about 8 to about 90 vol.-%, preferably in the range of about 15 to about 80 vol.-%, more preferably in the range of about 30 to about 70 vol.-%, even more preferably in the range of about 40 to about 60 vol.-%, such as bout 40, 45, 50, 55 or 60 vol.-%, based on the total volume of the biocomposite material.

In various embodiments, the biopolymer matrix component comprises the thiol-ene crosslinking reagent TMPTMP. The amount of thiol-ene crosslinking agent, which preferably is TMPTMP, may be in the range of about 10 to about 90 vol.-%, preferably in the range of about 20 to about 80 vol.-%, more preferably in the range of about 30 to about 70 vol.-%, even more preferably in the range of about 40 to about 60 vol.-%, such as bout 40, 45, 50, 55 or 60 vol.-%, based on the total volume of the biopolymer.

In various embodiments, the biopolymer matrix component comprises the catalyst DEA. The amount of catalyst, which preferably is DEA, may, in some embodiments, be in the range of about 1 to about 40 vol.-%, preferably in the range of about 2 to about 30 vol.-%, more preferably in the range of about 5 to about 20 vol.-%, even more preferably in the range of about 7 to about 15 vol.-%, such as bout 7,8,9,10,11,12,13,14 or 15 vol.-%, based on the total volume of the biopolymer.

In some embodiments of the present invention, the biopolymer matrix component further comprises at least one additional component, such as further additives that can commonly be used in the context of the present invention. According to various embodiments, the at least one further additive is selected from the group consisting of viscosity modifiers, photoinitiators, polymerization inhibitors, plasticizers, and radiopaque materials. Amounts of the aforementioned, non-limiting examples of further components suitable for employment according to the present invention are not particularly limited and may in the range of about 0.0001 to about 10 wt.-%, such as about 0.001 to about 5 wt.-%, based on the total weight of the biopolymer material herein described.

In various embodiments, the polymerization inhibitor is butylhydroxytoluene. In various embodiments, the polymerization inhibitor is comprised in an amount in the range of about 0.005 to about 2.5 wt.-%, preferably in an amount in the range of about 0.01 to about 1.0 wt.-%, more preferably in an amount in the range of about 0,01 to about 0.5 wt.-%, such as in an amount of about 0.01% by weight, based on the total weight of the biocomposite material. Thus, according to various embodiments, the polymerization inhibitor is butylhydroxytoluene and/or comprised in an amount of about 0.01% by weight, based on the total weight of the biocomposite material, to permit a long storage time and increase manipulation time.

According to various embodiments, the biopolymer matrix component is present in an amount of at least about 30 vol.-%, preferably at least about 40 vol.-%, preferably at least about 50 vol.-%, based on the total weight of the biocomposite material.

The biocomposite material of the present invention further comprises at least one bioactive filler component. The amount of said bioactive filler component may be in the range of about 10 to about 90 vol.-%, preferably in the range of about 20 to about 80 vol.-%, more preferably in the range of about 30 to about 70 vol.-%, even more preferably in the range of about 40 to about 60 vol.-%, such as bout 40, 45, 50, 55 or 60 vol.-%, based on the total volume of the biocomposite material. In various embodiments, the bioactive filler component is present in an amount of at least about 30 vol.-%, preferably at least about 40 vol.-%, preferably at least about 50 vol.-%, based on the total weight of the biocomposite material.

The bioactive filler may, according to various embodiments, be in the form of a powder, a particulate or granules. In various embodiments, it may comprise or consists of one or more components selected from the group consisting of Bioglass 45S5, vitamin D, vitamin B complex, and atorvastatin. The bioactive filler component, as herein described, may promote pulp regeneration (nerve and vessel) and promote mineralization and healing of dental structure.

In various embodiments, the bioactive filler component comprises or consists of bioactive glass, with, for instance but without limitation, silicon dioxide and calcium oxide as major constituents, and which enhances collagen mineralization and helps promote healing of hard tissue. According to preferred embodiments, the bioactive glass is a composition comprising or consisting of bioglass 45S5 (bio active glass for hard tissue regeneration) and/or Novamin (RTM). According to various embodiments, the bioactive glass may have the composition 46.1 mol% SiO₂, 24.4 mol% Na₂O, 26.9 mol% CaO-SiO₂ and 2.6 mol% P₂O₅. According to various embodiments, the bioactive filler component comprises the aforementioned composition or consists of the aforementioned composition.

The amount of bioglass may be in the range of about 5 to about 70 vol.-%, preferably in the range of about 10 to about 60 vol.-%, more preferably in the range of about 20 to about 50 vol.-%, even more preferably in the range of about 30 to about 40 vol.-%, such as bout 30, 31, 32, 33, 34, 35, 36, 37, 38, 39 or 40 vol.-%, based on the total volume of the bioactive filler component.

In various embodiments, the bioactive filler component comprises or consists of at least one nerve regenerative bioactive molecule. According to some embodiments, the bioactive molecule comprises or consists of Vitamin D (nerve regenerative molecule). The amount of Vitamin D may be in the range of about 5 to about 70 vol.-%, preferably in the range of about 10 to about 60 vol.-%, more preferably in the range of about 20 to about 50 vol.-%, even more preferably in the range of about 30 to about 40 vol.-%, such as bout 30, 31, 32, 33, 34, 35, 36, 37, 38, 39 or 40 vol.-%, based on the total volume of the bioactive filler component.

In various embodiments, the bioactive filler component comprises or consists of at least one nerve regenerative bioactive molecule. According to some embodiments, the bioactive molecule comprises or consists of Vitamin B complex (nerve regenerative). The amount of Vitamin B complex may be in the range of about 5 to about 70 vol.-%, preferably in the range of about 10 to about 60 vol.-%, more preferably in the range of about 20 to about 50 vol.-%, even more preferably in the range of about 30 to about 40 vol.-%, such as bout 30, 31, 32, 33, 34, 35, 36, 37, 38, 39 or 40 vol.-%, based on the total volume of the bioactive filler component.

In various embodiments, the bioactive filler component comprises or consists of at least one vascular regenerative bioactive molecule. According to some embodiments, the bioactive molecule comprises or consists of atorvastatin (a vascular regenerative molecule). The amount of atorvastatin may be in the range of about 5 to about 70 vol.-%, preferably in the range of about 10 to about 60 vol.-%, more preferably in the range of about 20 to about 50 vol.-%, even more preferably in the range of about 30 to about 40 vol.-%, such as bout 30, 31, 32, 33, 34, 35, 36, 37, 38, 39 or 40 vol.-%, based on the total volume of the bioactive filler component.

In various embodiments, the bioactive filler component, as herein described, comprises at least one additional component, such as further additives that commonly find application in the field of bone and dental (replacement) surgery, such as antibacterial agents, anti-inflammatory agents, cell proliferation inhibitors, antithrombotic agents, anticoagulants, neurotropic molecules, hormones, and further such bioactives. Amounts of the aforementioned, non-limiting examples of further components suitable for employment in the context of the present invention are not particularly limited and may in the range of about 0.0001 to about 10 wt.-%, such as about 0.001 to about 5 wt.-%, based on the total weight of the bioactive filler component herein described.

Thus, according to various embodiments, the biocomposite material of the present invention further comprises least one additional component selected from the group consisting of anti-inflammatory agents; antibiotics; enzymes inhibitors; hormones; cell growth inhibitors; anti-adhesion molecules; anticoagulants; and antithrombotic agents.

In some embodiments, the biocomposite material is in the form of a paste. In various embodiments, the viscosity of the biocomposite material according to the present invention is in the range of about 10⁴ to 10¹² mPa·s. The viscosity may be chosen and adapted based on varying clinical needs and thus varying clinical implementation of the biocomposite material of the present invention.

The present invention further relates to a method for manufacturing a biocomposite material as herein disclosed and described. Generally, the method comprises a step a) of mixing together the at least one bioactive filler component and the at least one biopolymer matrix component.

According to various embodiments, the bioactive filler component is in the form of a powder, particulate or granules and the biopolymer matrix component is in liquid form, and both components are mixed together in step a) of the herein described method.

In various embodiments, the biopolymer matrix components and the bioactive fillers components are, independently of each other, present in the mixture obtained in step a) in an amount of about 10 to about 90 vol.-%, preferably in an amount of about 20 to about 80 vol.-%, preferably in an amount of about 30 to about 70 vol.-%, more preferably in an amount of about 40 to about 60 vol.-%, based on the total volume of said mixture. According to preferred embodiments, said components are present in the mixture obtained in step a) in equal amounts.

According to various embodiments, the biopolymer matrix component comprises a thio-acrylate polymer and at least one further component selected from the group consisting of viscosity modifiers, photoinitiators, polymerization inhibitors, and plasticizers, wherein said at least one further component is present in an amount in the range of about 1 to 10 vol.-%, based on the total volume of the biopolymer matrix component.

According to various embodiments, the herein described method comprises at least one further step b) of adding to the mixture obtained in step a) one or more additives selected from the group consisting of anti-inflammatory agents; antibiotics; enzymes inhibitors; hormones; cell growth inhibitors; anti-adhesion molecules; anticoagulants; and antithrombotic agents.

Preferably, the biocomposite material of the present invention should be provided/presented/stored in an opaque tube holding the biocomposite in pre-prepared paste form. The biocomposite material may be also presented in form of a powder mixture and polymer liquid components to be mixed immediately before use of the biocomposite material, or in the form of two different pastes (i.e. a base and an activator paste, a base comprising at least one triacrylate monomer, as herein defined, and an activator comprising the at least one crosslinking agent and the at least one catalyst, as herein defined) to be freshly mixed prior to application of the biocomposite material.

Thus, in a further aspect, the present invention also relates to a kit-of-parts comprising at least one biopolymer matrix component, as herein defined and described above, at least one bioactive filler component, as herein defined and described above, and optionally further at least one UV light source and/or at least one opaque, preferably sealable container suitable for accommodating a biocomposite material as herein described above and/or or obtainable in a method, as herein described above,
In various embodiments, the biopolymer matrix components and the bioactive fillers components are each presented in a closed, preferably opaque container. A UV light source may be included and be used to activate polymer formation and initiate/promote hardening.

The biocomposite material herein defined and described may find application in many different clinical scenarios, including, without limitation, biopulptomy (temporary and permanent teeth), pulp capping (direct or indirect), also for temporary and for permanent teeth, as well as in treating floor perforation of the tooth or root perforation, and last but not least as obturation material for endodontic surgery and retrograde obturation. Thus, in a further aspect, the present invention also relates to the use of a biocomposite material as herein defined and described, or a biocomposite material obtainable in a method as herein described in biopulpectomy procedures for temporary or permanent teeth, in direct or indirect pulp capping, as a retrograde filling material for endodontic surgery, or for tooth or root perforation.

The present invention is further directed to a method for obtaining a hardened biocomposite material, the method comprising the steps of:
i) providing a biocomposite material, as herein defined and described above, or a biocomposite material obtainable in a method as herein described above; and
ii) exposing said biocomposite material provided in step i) to electromagnetic radiation, preferably Ultraviolet (UV), more preferably UVA, more preferably UV having a wavelength of about 365 nm to obtain a hardened composite material.

Times of exposure may be in the range of about 0.1 sec to about 13 min, preferably in the range of about 5 sec to about 10 min, more preferably in the range of about 30 sec to about 5 min, such as about 30 sec, 1 min, 1.5 min, 2 min, 2.5 min, 3, min, 3.5 min, 4 min, 4.5 min, or 5 min.

### EXAMPLES

### Example 1: Example of biocomposite application

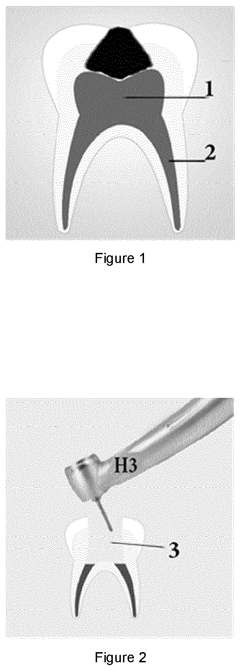

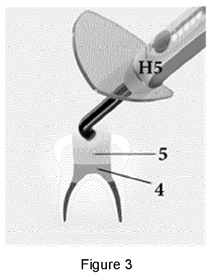

After local anesthesia of the tooth, the caries was removed as well as a part of the pulp (coronal part = biopulpotomy - number 1 in Figure 1), resulting in tooth cavity (Figure 2), then the biocomposite material (presented in the form of a pre-prepared paste) is placed at the base of the cavity and in direct contact to the remaining vital pulp of the tooth (figure 3), then UV light source (blue light indicated in Figure 3) is applied in order to initiate material hardening and polymerization (Figure 3).

## Claims

1. Biocomposite material comprising at least one biopolymer matrix component and at least one bioactive filler component.

2. The biocomposite material according to claim 1, **characterized in that** the biopolymer matrix component comprises or consists of at least one thiol-triacrylate.

3. The biocomposite material according to claim 1 or 2, **characterized in that** the thiol-triacrylate polymer is obtainable by reacting at least one triacrylate monomer, at least one thiol-ene crosslinking agent, and at least one catalyst.

4. The biocomposite material according to any one of claims 1 to 3, **characterized in that** the triacrylate monomer is selected from the group consisting of TMPTA (trimethylolpropane triacrylate), PETA (pentaerythritol triacrylate), TMPeTA (trimethylolpropane ethoxylate triacrylate), and TMPPTA (trimethylolpropane propoxylate triacrylate).

5. The biocomposite material according to any one of claims 1 to 4, **characterized in that**
- the at least one crosslinking reagent is trimethylolpropane tris (3-mercaptopropionate); and/or
- the at least one catalyst is diethylamine; and/or
- the biopolymer matrix component further comprises at least one additional component selected from the group consisting of viscosity modifiers, photoinitiators, polymerization inhibitors, plasticizers, and radiopaque materials.

6. The biocomposite material according to claim 5, **characterized in that** the polymerization inhibitor is butylhydroxytoluene and/or comprised in an amount of about 0.01% by weight, based on the total weight of the biocomposite material.

7. The biocomposite material according to any one of claims 1 to 6, **characterized in that** the bioactive filler component comprises or consists of one or more components selected from the group consisting of Bioglass 45S5, vitamin D, vitamin B complex, and atorvastatin.

8. The biocomposite material according to any one of claims 1 to 7, **characterized in that**
- the biopolymer matrix component is present in an amount of at least about 30 vol.-%, preferably at least about 40 vol.-%, preferably at least about 50 vol.-%, based on the total weight of the biocomposite material; and/or
- the bioactive filler component is present in an amount of at least about 30 vol.-%, preferably at least about 40 vol.-%, preferably at least about 50 vol.-%, based on the total weight of the biocomposite material; and/or
- the biocomposite material is in the form of a paste.

9. The biocomposite material according to any one of claims 1 to 8, **characterized in that** it further comprises least one additional component selected from the group consisting of anti-inflammatory agents; antibiotics; enzymes inhibitors; hormones; cell growth inhibitors; anti-adhesion molecules; anticoagulants; and antithrombotic agents.

10. Method for manufacturing a biocomposite material according to any one of claims 1 to 9, comprising the step a) of mixing together the at least one bioactive filler component and the at least one biopolymer matrix component.

11. The method according to claim 10, **characterized in that**
- the bioactive filler component is in particulate form and the biopolymer matrix component is in liquid form; and/or
- the biopolymer matrix component comprises a thio-acrylate polymer and at least one further component selected from the group consisting of viscosity modifiers, photoinitiators, polymerization inhibitors, and plasticizers, wherein said at least one further component is present in an amount in the range of about 1 to 10 vol.-%, based on the total volume of the biopolymer matrix component; and/or
- the at least one bioactive filler component and the at least one biopolymer matrix component are mixed in equal proportions.

12. Method for obtaining a hardened biocomposite material, the method comprising the steps of:
i) providing a biocomposite material according to any one of claims 1 to 9 or obtainable in a method according to claim 10 or 11; and
ii) exposing said biocomposite material to electromagnetic radiation, preferably Ultraviolet (UV), more preferably UVA, more preferably UV having a wavelength of about 365 nm to obtain a hardened biocomposite material.

13. Use of a biocomposite material according to any one of claims 1 to 9 or obtainable in a method according to claim 10 or 11 in biopulpectomy procedures for temporary or permanent teeth, in direct or indirect pulp capping, as a retrograde filling material for endodontic surgery, or for tooth or root perforation.

14. Kit-of-parts, comprising at least one biopolymer matrix component as defined in any one of claims 2 to 6, at least one bioactive filler component as defined in claim 7, and optionally further at least one UV light source and/or at least one opaque, preferably sealable container suitable for accommodating a biocomposite material according to anyone of claims 1 to 9 or obtainable in a method according to claim 10 or 11.
